# EUROPEAN PATENT APPLICATION

(11) **EP 1 813 684 A2**
(43) Date of publication of application: **01.08.2007**
(21) Application number: 07100727.2
(22) Date of filing: 14.02.1995
(51) Int. Cl.: C12Q 1/68

(54) **Differently expressed genes in healthy and diseased subjects**

(30) Priority: 14.02.1994 US 195485
(62) Divisional of application: 95910257.5
(71) Applicant: SMITHKLINE BEECHAM CORPORATION, King of Prussia, PA 19406-0939 (US)
(72) Inventor: Rosenberg, Martin, Royersford, PA 19468 (US); Debouck, Christine, Wayne, PA 19087 (US); Bergsma, Derk, Berwyn, PA 19312 (US)
(74) Representative: Priestley, Ruth Elizabeth

(57) **Abstract**

The present invention involves methods and compositions for identifying genes which are differentially expressed in a normal healthy animal and an animal having a selected disease or infection, and methods for diagnosing diseases or infections characterized by the presence of those genes, despite the absence of knowledge about the gene or its function. The methods involve the use of a composition suitable for use in hybridization which consists of a solid surface on which is immobilized at pre-defined regions thereon a plurality of defined oligonucleotide/polynucleotide sequences for hybridization. Each sequence comprises a fragment of an EST isolated from an identified DNA library prepared from tissue or cell samples of a healthy animal, an animal with a selected disease or infection, and any combination thereof. Differences in hybridization patterns produced through use of this composition and the specified methods enable diagnosis of disease based on differential expression of genes of unknown function, and enable the identification of those genes and the proteins encoded thereby.

## Description

### Cross Reference to Related Applications:

This application is a continuation-in-part application of U.S. Serial No. 08/195,485 filed February 14, 1994, the contents of which are incorporated herein by reference.

### Field of the Invention

The present invention relates to the use of immobilized oligonucleotide/polynucleotide or polynucleotide sequences for the identification, sequencing and characterization of genes which are implicated in disease, infection, or development and the use of such identified genes and the proteins encoded thereby in diagnosis, prognosis, therapy and drug discovery.

### Background of the Invention

Identification, sequencing and characterization of genes, especially human genes, is a major goal of modem scientific research. By identifying genes, determining their sequences and characterizing their biological function, it is possible to employ recobinant DNA technology to produce large quantities of valuable "gene products", e.g., proteins and peptides. Additionally, knowledge of gene sequences can provide a key to diagnosis, prognosis and treatment of a variety of disease states in plants and animals which are characterized by inappropriate expression and/or repression of selected gene(s) or by the influence of external factors, e.g., carcinogens or teratogens, on gene function. The term disease-associated genes(s) is used herein in its broadest sence to mean not only genes associated with classical inherited diseases, but also those associated with genetic predisposition to disease as well as infectious or pathogenic states resulting from gene expression by infectious agents or the effect on host cell gene expression by the presence of such a pathogen or its products Locating disease-associated genes will permit the development of diagnostic and prognostic reagents and methods, as well as possible therapeutic regimens, and the discovery of new drugs for treating or preventing the occurrence of such diseases.

Methods have been described for the identification of certain novel gene sequences, referred to as Expressed Sequence Tags (EST) [see, e.g., Adams et al, Science, 252:1651-1656 (1991); and International Patent Application No. WO93/00353, published January 7, 1993]. Conventially, an EST is a specific cDNA polynucleotide sequence, or tag, about 150 to 400 nucleotides in length, derived from a messenger RNA molecule by reverse transcription, which is a marker for, and component of, a human gene actually transcribed *in vivo*. However, as used herein an EST also refers to a genomic DNA fragment derived from an organism, such as a microorganism,the DNA of which lacks intron regions.

A variety of techniques have been described for identifying particular gene sequences on the basis of their gene products. For example, several techniques are described in the art [see, e.g., International Patent Application No. WO91/07087, published May 30, 1991]. Additionally, known methods exist for the amplification of desired sequences [see, e.g., International Patent Application No. WO91/17271, published November 14,1991, among others].

However, at present, there exist no established methods for filling the need in the art for methods and reagents which employ fragments of differentially expressed genes of known, unknown (or previously unrecognized) function or consequence to provide diagnostic and therapeutic methods and reagents for diagnosis and treatment of disease or infection, which conditions are characterized by such genes and gene products. It should be appreciated that it is the expression differences that are diagnostic of the altered state (e.g., predisease, disease, pathogenic, progression or infectious). Such genes associated with the altered state are likely to be the targets of drug discovery, whether the genes are the cause or the effect of the condition, identification of such genes provides insight into which gene expression needs to be re-altered in order to reestablished the healthy state.

### Summary of the Invention

In one aspect, the invention provides methods for identifying gene(s) which are differentially expressed, for example, in a normal healthy organism and an organism having a disease. The method involves producing and comparing hybridization patterns formed between samples of expressed mRNA or cDNA polynucleotide sequences obtained from either analogous cells, tissues or organs of a healthy organism and a diseased organism and a defined set of oligonucleotide/polynucleotide/polynucleotide sequence probes from either an healthy organism or a diseased organism immobilized on a support. Those defined oligonucleotide/polynucleotide sequences are representative of the total expressed genetic component of the cells, tissues, organs or organism as defined the collection of partial cDNA sequences (ESTs). The differences between the hybridization patterns permit identification of those particular EST or gene-specific oligonucleotide/polynucleotide sequences associated with differential expression, and the identification of the EST permits identification of the clone from which it was derived and using ordinary skill further cloning and, if desired, sequencing of the full-length cDNA and genomic counterpart, i.e., gene, from which it was obtained.

In another aspect, the invention provides methods substantially similar to those described above, but which permit identification of those gene(s) of a pathogen which are expressed in any biological sample of an infected organism based on comparative hybridization of RNA/cDNA samples derived from a healthy versus infected organism, hybridized to an oligonucleotide/polynucleotide set representative of the gene coding complement of the pathogen of interest.

In another aspect, the invention provides methods substantially similar to those described above, but which permit identification of those ESTs-specific oligonucleotide/polynucleotide sequences of host gene(s) which represent genes being differentially expressed/ altered in expression by the disease state, or infection and are expressed in any biological sample of an infected organism based on comparative hybridization of RNA/cDNA samples derived from a healthy versus infected organism of interest.

In a further aspect, the methods described above and in detail below, also provide methods for diagnosis of diseases or infections characterized by differentially expressed genes, the expression of which has been altered as a result of infection by the pathogen or disease causing agent in question. All identified differences provide the basis for diagnostic testing be it the altered expression of endogenous genes or the patterned expression of the genes of the infecting organism. Such patterns of altered expression are defined by comparing RNA/cDNA from the two states hybridized against a panel of oligonuclcotide/polynucleotides representing the expressed gene component of a cell, tissue, organ or organism as defined by its collection of ESTs.

Yet a further aspect of this invention provides a composition suitable for use in hybridization, which comprises a solid surface on which is immobilized at pre-defined regions thereon a plurality of defined oligonucleotide/polynucleotide sequences for hybridization, each sequence comprising a fragment of an EST isolated from a cDNA or DNA library prepared from at least one selected tissue or cell sample of a healthy (i.e., pre-disease state) animal, at least one analogous sample of an animal having a disease, at least one analogous sample of an animal infected with a pathogen or the pathogen itself, or any combination or multiple combinations thereof.

An additional aspect of the invention provides an isolated gene sequence which is differentially expressed in a normal healthy animal and an animal having a disease, and is identified by the methods above. Similarly, an isolated pathogen gene sequence which is expressed in tissue or cell samples of an infected animal can be identified by the methods above.

Yet another aspect of the invention is that it provides not only a means for a static diagnostic but also provides a means for a carrying out the procedure over time to measure disease progression as well as monitoring the efficacy of disease treatment regimes including an toxicological effects thereof.

Another aspect of the invention is an isolated protein produced by expression of the gene sequences identified above. Such proteins are useful in therapeutic compositions or diagnostic compositions, or as targets for drug development.

Other aspects and advantages of the present invention are described further in the following detailed description of the preferred embodiments thereof.

### Detailed Description of the Invention

The present invention meets the unfulfilled needs in the art by providing methods for the identification and use of gene fragments and genes, even those of unknown full length sequence and unknown function, which are differentially expressed in a healthy animal and in an animal having a specific disease or infection by use of ESTs derived from DNA libraries of healthy and/or diseased/infected animals. Employing the methods of this invention permits the resulting identification and isolation of such genes by using their corresponding ESTs and thereby also permits the production of protein products encoded by such genes. The genes themselves and/or protein products, if desired, may be employed in the diagnosis or therapy of the disease or infection with which the genes are associated and in the development of new drugs therefor.

It has been appreciated that one or more differentially identified EST or gene-specific oligonucleotide/polynucleotides define a pattern of differentially expressed genes diagnostic of a predisease, disease or infective state. A knowledge of the specific biological function of the EST is not required only that the ESTs identifies a gene or genes whose altered expression is associated reproducibly with the predisease, disease or infectious state. The differences permit the identification of gene products altered in their expression by the disease and represent those products most likely to be targets of therapeutic intervention. Similarly, the product may be of the infecting organism itself and also be an effective target of intervention.

### I. Definitions.

Several words and phrases used throughout this specification are defined as follows:

As used herein, the term "gene" refers to the genomic nucleotide sequence from which a cDNA sequence is derived, which cDNA produces an EST, as described below. The term gene classically refers to the genomic sequence, which, upon processing, can produce different cDNAs, e.g., by splicing events. However, for ease of reading, any full-length counterpart cDNA sequence which gives rise to an EST will also be referred to by shorthand herein as a 'gene'.

The term "organism" includes without limitation, microbes, plants and animals.

The term "animal" is used in its broadest sense to include all members of the animal kingdom, including humans. It should be understood, however, that according to this invention the same species of animal which provides the biological sample also is the source of the defined immobilized oligonucleotide/polynucleotides as defined below.

The term "pathogen" is defined herein as any molecule or organism which is capable of infecting an animal or plant and replicating its nucleic acid sequences in the cells or tissues of that animal or plant. Such a pathogen is generally associated with a disease condition in the infected animal or plant. Such pathogens may include viruses, which replicate intra- or extra-cellularly, or other organisms, such as bacteria, fungi or parasites, which generally infect tissues or the blood. Certain pathogens or microorganisms are known to exist in sequential and distinguishable stages of development, e.g., latent stages, infective stages, and stages which cause symptomatic diseases. In these different stages, the pathogens are anticipated to express differentially certain genes and/or turn on or off host cell gene expression.

As used herein, the term "disease" or "disease state" refers to any condition which deviates from a normal or standardized healthy state in an organism of the same species in terms of differential expression of the organism's genes. In other words, a disease state can be any illness or disorder be it of genetic or environmental origin , for example, an inherited disorder such as certain breast cancers, or a disorder which is characterized by expression of gene(s) normally in an inactive, 'turned off' state in a healthy animal, or a disorder which is characterized by under-expression or no expression of gene(s) which is normally activated or 'turned on' in a normal healthy animal. Such differential expression of genes may also be detected in a condition caused by infection, inflammation, or allergy, a condition caused by development or aging of the animal, a condition caused by administration of a drug or exposure of the animal to another agent, e.g., nutrition, which affects gene expression. Essentially, the methods described herein can be adapted to detect differential gene expression resulting from any cause, by manipulation of the defined oligonucleotide/polynucleotides and the samples tested as described below. The concept of disease or disease state also includes its temporal aspects in terms of progression and treatment.

The phrase "differentially expressed" refers to those situations in which a gene transcript is found in differing numbers of copies, or in activated vs inactivated states, in different cell types or tissue types of an organism, having a selected disease as contrasted to the levels of the gene transcript found in the same cells or tissues of a healthy organism. Genes may be differentially expressed in differing states of activation in microorganisms or pathogens in different stages of development. For example, multiple copies of gene transcripts may be found in an organism having a selected disease, while only one, or significantly fewer copies, of the same gene transcript are found in a healthy organism, or vice-versa.

As used herein, the term "solid support" refers to any known substrate which is useful for the immobilization of large numbers of oligonucleotide/polynucleotide sequences by any available method to enable detectable hybridization of the immobilized oligonucleotide/polynucleotide sequences with other polynucleotide sequences in a sample. Among a number of available solid supports, one desirable example is the supports described in International Patent Application No. WO91/07087, published May 30, 1991.Also useful are suports such as but not limited to nitrocellulose, mylein, glass, silica ans Pall Biodyne C^{®} It is also anticipated that improvements yet to be made to conventional solid supports may also be employed in this invention.

The term "surface" means any generally two-dimensional structure on a solid support to which the desired oligonucleotide/polynucleotide sequence is attached or immobilized. A surface may have steps, ridges, kinks, terraces and the like.

As used herein, the term "predefined region" refers to a localized area on a surface of a solid support on which is immobilized one or multiple copies of a particular oligonucleotide/polynucleotide sequence and which enables the identification of the oligonucleotide/polynucleotide at the position, if hybridization of that oligonucleotide/polynucleotide to a sample polynucleotide occurs.

By "immobilized" refers to the attachment of the oligonucleotide/polynucleotide to the solid support. Means of immobilization are known and conventional to those of skill in the art, and may depend on the type of support being used.

By "EST" or "Expressed Sequence Tag" is meant a partial DNA or cDNA sequence of about 150 to 500, more preferably about 300, sequential nucleotides of a longer sequence obtained from a genomic or cDNA library prepared from a selected cell, cell type, tissue or tissue type, organ or organism which longer sequence corresponds to an mRNA of a gene found in that library. An EST is generally DNA. One or more libraries made from a single tissue type typically provide at least about 3000 different (i.e., unique) ESTs and potentially the full complement of all possible ESTs representing all cDNAs e.g., 50,000-100,000 in an animal such as a human. Further background and information on the construction of ESTs is described in M. D. Adams et al, Science, 252:1651-1656 (1991); and International Application Number PCT/US92/05222 (January 7, 1993).

As used herein, the term "defined oligonucleotide/polynucleotide sequence" refers to a known nucleotide sequence fragment of a selected EST or gene. This term is used interchangeably with the term "fragments of EST". These sequential sequences are generally comprised of between about 15 to about 45 nucleotides and more preferably between about 20 to about 25 nucleotides in length. Thus any single EST of 300 nucleotides in length may provide about 280 different defined oligonucleotide/polynucleotide sequences of 20 nucleotides in length (e.g., 20-mers). The lengths of the defined oligonucleotide/polynucleotides may be readily increased or decreased as desired or needed, depending on the limitations of the solid support on which they may be immobilized or the requirements of the hybridization conditions to be employed.The length is generally guided by the principle that it should be of sufficient length to insure that it is one average only represented once in the population to be examined. Generally, these defined oligonucleotide/polynucleotides are RNA or DNA and are preferably derived from the anti-sense strand of the EST sequence or from a corresponding mRNA sequence to enable their hybridization with samples of RNA or DNA. Modified nucleotides may be incorporated to increase stability and hybridization properties.

By the term "plurality of defined oligonucleotide/polynucleotide sequences" is meant the following. A surface of a solid support may immobilize a large number of "defined oligonucleotide/polynucleotides". For example, depending upon the nature of the surface, it can immobilize from about 300 to upwards of 60,000 defined 20-mer oligonucleotide/polynucleotides. It is anticipated that future improvements to solid surfaces will permit considerably larger such pluralities to be immobilized on a single surface. A "plurality" of sequences refers to the use on any one solid support of multiple different defined oligonucleotide/polynucleotides from a single EST from a selected library, as well as multiple different defined oligonucleotide/polynucleotides from different ESTs from the same library or many libraries from the same or different tissues, and may also include multiple identical copies of defined oligonucleotide/polynucleotides. Ultimately a pluarality has at least one oligonucleotide/polynucleotide per expressed gene in the entire organism For example, from a library producing about 5,000-10,000 ESTs, a single support can include at least about 1-20 defined oligonucleotide/polynucleotides representing every EST in that library. The composition of defined oligonucleotide/polynucleotides which make up a surface according to this invention may be selected or designed as desired.

The term "sample" is employed in the description of this invention in several important ways. As used herein, the term "sample" encompasses any cell or tissue from an organism. Any desired cell or tissue type in any desired state may be selected to form a sample. For example, the sample cell desired may be a human T cell; the desired cell type for use in this invention may be a quiescent T cell or an activated T cell.

By the phrase "analogous sample" or "analogous cell or tissue" is meant that according to this invention when the ESTs which provide the defined oligonucleotide/polynucleotides are produced from a cDNA library prepared from a single tissue or cell type source sample, e.g., liver tissue of a human, then the samples used to hybridize to those immobilized defined oligonucleotide/polynucleotides are preferably provided by the same type of sample from either a healthy or diseased animal, i.e., liver tissue of a healthy human and liver tissue of a diseased or infected human or from a human suspected of having that disease or infection. Alternatively, if the surface contains defined oligonucleotide/polynucleotides from multiple cells or tissues, then the "samples" which are hybridized thereto can be but are not limited to samples obtained from analogous multiple tissues or cells.

By the term "detectably hybridizing" means that the sample from the healthy organism or diseased or infected organism is contacted with the defined oligonucleotide/polynucleotides on the surface for sufficient time to permit the formation of patterns of hybridization on the surfaces caused by hybridization between certain polynucleotide sequences in the samples with the certain immobilized defined oligonucleotide/polynucleotides. These patterns are made detectable by the use of available conventional techniques, such as fluorescent labelling of the samples. Preferably hybridization takes place under stringent conditions, e.g., revealing homologies of about 95%. However, if desired, other less stringent conditions may be selected. Techniques and conditions for hybridization at selected stringencies are well known in the art [see, e.g., Sambrook et al, Molecular Cloning, A Laboratory Manual., Cold Spring Harbor Laboratory, Cold Spring Harbor, NY (1989)].

### II. Compositions of The Invention

The present invention is based upon the use of ESTs from any desired cell or tissue in known technologies for oligonucleotide/polynucleotide hybridization.

### A. ESTs

An EST, as defined above, is for an animal, a sequence from a cDNA clone that corresponds to an mRNA. The EST sequences useful in the present invention are isolated preferably from cDNA libraries using a rapid screening and sequencing technique. Custom made cDNA libraries are made using known techniques. See, generally, Sambrook et al, cited above. Briefly, mRNA from a selected cell or tissue is reverse transcribed into complementary DNA (cDNA) using the reverse transcriptase enzyme and made double-stranded using RNase H coupled with DNA polymerase or reverse transcriptase. Restriction enzyme sites are added to the cDNA and it is cloned into a vector. The result is a cDNA library. Alternatively, commercially available cDNA libraries may be used. Libraries of cDNA can also be generated from recombinant expression of genomic DNA using known techniques, including polymerase chain reaction-derived techniques.

ESTs (which can range from about 150 to about 500 nucleotides in length, preferably about 300 nucleotides) can be obtained through sequence analysis from either end of the cDNA insert. Desirably, the DNA libraries used to obtain ESTs use directional cloning methods so that either the 5' end of the cDNA (likely to contain coding sequence) or the 3' end (likely to be a non-coding sequence) can be selectively obtained.

In general, the method for obtaining ESTs comprises applying conventional automated DNA sequencing technology to screen clones, advantageously randomly selected clones, from a cDNA library. The cDNA libraries from the desired tissue can be preprocessed, or edited, by conventional techniques to reduce repeated sequencing of high and intermediate abundance clones and to maximize the chances of finding rare messages from specific cell populations. Preferably, preprocessing includes the use of defined composition prescreening probes, e.g., cDNA corresponding to mitochondria, abundant sequences, ribosomes, actins, myelin basic polypeptides, or any other known high abundance peptide. These prescreening probes used for preprocessing are generally derived from known ESTs. Other useful preprocessing techniques include subtraction hybridization, which preferentially reduces the population of highly represented sequences in the library [e.g., see Fargnoli et al, Anal. Biochem., 187:364 (1990)] and normalization, which results in all sequences being represented in approximately equal proportions in the library [Patanjali et al, Proc. Natl. Acad. Sci. USA, 88:1943 (1991)]. Additional prescreening/differential screening approaches are known to those skilled in the art.

ESTs can then be generated from partial DNA sequencing of the selected clones. The ESTs useful in the present invention are preferably generated using low redundancy of sequencing, typically a single sequencing reaction. While single sequencing reactions may have an accuracy as low as 90%, this nevertheless provides sufficient fidelity for identification of the sequence and design of PCR primers.

If desired, the location of an EST in a full length cDNA is determined by analyzing the EST for the presence of coding sequence. A conventional computer program is used to predict the extent and orientation of the coding region of a sequence (using all six reading frames). Based on this information, it is possible to infer the presence of start or stop codons within a sequence and whether the sequence is completely coding or completely non-coding or a combination of the two. If start or stop codons are present, then the EST can cover both part of the 5'-untranslated or 3'-untranslated part of the mRNA (respectively) as well as part of the coding sequence. If no coding sequence is present, it is likely that the EST is derived from the 3' untranslated sequence due to its longer length and the fact that most cDNA library construction methods are biased toward the 3' end of the mRNA. It should be understood that both coding and non-coding regions may provide ESTs equally useful in the described invention.

A number of specific ESTs suitable for use in the present invention are described above Adams et al (supra), which may be incorporated by reference herein, to describe non-essential examples of desirable ESTs. Other ESTs exist in the art which may also be useful in this invention, as will ESTs yet to be developed by these known techniques.

### B. Preparing the Solid Support of the Invention

Oligonucleotide sequences which are fragments of defined sequence are derived from each EST by conventional means, e.g., conventional chemical synthesis or recombinant techniques. Each defined oligonucleotide/polynucleotide sequence as described above is a fragment, can be, but is not necessarily an anti-sense fragment, of an EST isolated from a DNA library prepared from a selected cell or tissue type from a selected animal. For use in the present invention, it is presently preferred that the defined oligonucleotide/polynucleotide sequences are 20-25mers. As described above, for each EST a number of such 20-25mers may be generated. The lengths may vary as described above as well as the composition. For example oligonucleotide/polynucleotides can be modified based on the Oligo 4.0 or simiolar programs to predict hybridization potential or to include modifieid nucleotides for the reasons given above. It is alos appreciated that large DNA segments may be employed including entire ESTs or even full length genes particular when inserted into cloning vectors.

A plurality of these defined oligonucleotide/polynucleotide sequences are then attached to a selected solid support conventionally used for the attachment of nucleotide sequences again by known means. In contrast to other technologies available in the art, this support is designed to contain defined, not random, oligonucleotide/polynucleotide sequences. The EST fragments, or defined oligonucleotide/polynucleotide sequences, immobilized on the solid support can include fragments of one or more ESTs from a library of at least one selected tissue or cell sample of a healthy animal, at least one analogous sample of the animal having a disease, at least one analogous sample of the animal infected with a pathogen, and any combination thereof.

Numerous conventional methods are employed for attaching biological molecules such as oligonucleotide/polynucleotide sequences to surfaces of a variety of solid supports. See, e.g., Affinity Techniques. Enzyme Purification: Part B. Methods in Enzymology, Vol. 34, ed. W.B. Jakoby, M. Wilcheck, Acad. Press, NY (1974); Immobilized Biochemicals and Affinity Chromatography, Advances in Experimental Medicine and Biology, vol. 42, ed. R. Dunlap, Plenum Press, NY (1974); U. S. Patent No. 4,762,881; U. S. Patent No. 4,542,102; European Patent Publication No. 391,608 (October 10, 1990); U. S. Patent No. 4,992,127 (Nov. 21, 1989).

One desirable method for attaching oligonucleotide/polynucleotide sequences derived from ESTs to a solid support is described in International Application No. PCf/US90/06607 (published May 30, 1991). Briefly, this method involves forming predefined regions on a surface of a solidsupport, where the predefined regions are capable of immobilizing ESTs. The methods make use of binding substances attached to the surface which enable selective activation of the predefined regions. Upon activation, these binding substances become capable of binding and immobilizing oligonucleotide/polynucleotides based on EST or longer gene sequences.

Any of the known solid substrates suitable for binding oligonucleotide/polynucleotides at pre-defined regions on the surface thereof for hybridization and methods for attaching the oligonucleotide/polynucleotides thereto may be employed by one of skill in the art according to this invention. Similarly, known conventional methods for making hybridization of the immobilized oligonucleotide/polynucleotides detectable, e.g., fluorescence, radioactivity, photoactivation, biotinylation, solid state circuitry, and the like may be used in this invention.

Thus, by resorting to known techniques, the invention provides a composition suitable for use in hybridization which consists of a surface of a solid support on which is immobilized at pre-defined regions on said surface a plurality of defined oligonucleotide/polynucleotide sequences for hybridization. For example, one composition of this invention is a solid support on which are immobilized oligos of EST fragments from a library constructed from a single cell type, e.g., a human stem cell, or a single tissue, e.g., human liver, from a healthy human. Still another composition of this invention is another solid support on which are immobilized oligos of EST fragments from a library constructed from a single cell type or a tissue from a human having a selected disease or predispositon to a selected disease, e.g., liver cancer.

Another embodiment of the compositions of this invention include a single solid support having oligonucleotides of ESTs from both single cell or single tissue libraries from both a healthy and diseased human. Still other embodiments include a single support on which are immobilized oligos of EST fragments from more than one tissue or cell library from a healthy human or a single support on which are immobilized more than one tissue or cell library from both healthy and diseased animals or humans. A preferred composition of this invention is anticipated to be a single support containing oligos of ESTs for all known cells and tissues from a selected organism.

### III. The Methods of the Invention

### A. Identification of Genes

The present invention employs the compositions described above in methods for identifying genes which are differentially expressed in a normal healthy organism and an organism having a disease or infection. These methods may be employed to detect such genes, regardless of the state of knowledge about the function of the gene. The method of this invention by use of the compositions containing multiple defined EST fragments from a single gene as described above is able to detect levels of expression of genes or in other cases simply the expression or lack thereof, which differ between normal, healthy organisms and organisms having a selected disease, disorder or infection.

One such method employs a first surface of a solid support on which is immobilized at pre-defined regions thereon a plurality of defined oligonucleotide/polynucleotide sequences, described above, of ESTor longer gene fragment isolated from a cDNA library prepared from at least one selected tissue or cell sample of a healthy animal (the "healthy test surface") and a second such surface on which is immobilized at pre-defined regions a plurality of defined oligonucleotide/polynucleotide sequences of ESTor longer gene fragment isolated from at least one analogous tissue of an animal having a selected disease (the "disease test surface"). These test surfaces may be standardized for the selected animal or selected cell or tissue sample from that animal (i.e., they are prescreened for polymorphisms in the species population).

Polynucleotide sequences are then isolated from mRNA and/or cDNA from a biological sample from a known healthy animal ("healthy control") and a second sample is similarly prepared from a sample from a known diseased animal ("disease sample"). These two samples are desirably selected from the cell or tissue analogous to that which provided the immobilized oligonucleotide/polynucleotides.

According to the method the healthy control sample is contacted with one set of the healthy test surface and the disease test surface described above for a time sufficient to permit detectable hybridization to occur between the sample and the immobilized defined oligonucleotide/polynucleotides on each surface. The results of this hybridization are a first hybridization pattern formed between the nucleotides of healthy control and the healthy test surface and a second hybridization pattern formed between the nucleotides of healthy control sample and the disease test surface.

In a similar manner, the disease sample is detectably hybridized to another set of healthy test and disease test surfaces, forming a third hybridization pattern between the disease sample and healthy test surface and a fourth hybridization pattern between the disease sample and the disease test surface.

Comparing the four hybridization patterns permits detection of those defined oligonucleotide/polynucleotides which are differentially expressed between the healthy control and the disease sample by the presence of differences in the hybridization patterns at pre-defined regions. The oligonucleotide/polynucleotides on each surface which correspond to the pattern differences may be readily identified with the corresponding ESTor longer gene fragment from which the oligonucleotide/polynucleotides are obtained.

In another embodiment of the method of this invention, the same process is employed, with the exception that plurality of defined oligonucleotide/polynucleotide sequences forming the healthy test sample and the disease test sample surfaces are immobilized on a single solid support. For example, each fragment of an EST or longer gene fragment on the surface is isolated from at least two cDNA libraries prepared from a selected cell or tissue sample of a healthy animal and an analogous selected cell or tissue sample of an animal having a disease.

According to this embodiment, the healthy control sample is detectably hybridized to a copy of this single solid surface, forming one hybridization pattern with oligonucleotide/polynucleotides associated with both the healthy and diseased animal. Similarly, the disease sample is detectably hybridized to a second copy of this single solid surface, forming one hybridization pattern with oligonucleotide/polynucleotides associated with both the healthy and diseased animal.

Comparing the two hybridization patterns permits detection of those defined oligonucleotide/polynucleotides which are differentially expressed between the healthy control and the disease sample by the presence of differences in the hybridization patterns at pre-defined regions. The oligonucleotide/polynucleotides on each surface which correspond to the pattern differences may be readily identified with the corresponding ESTor longer gene fragment from which the oligonucleotide/polynucleotides are obtained.

The identification of one or more ESTs as the source of the defined oligonucleotide/polynucleotide which produced a "difference" in hybridization patterns according to these methods permits ready identification of the gene from which those ESTs were derived. Because oligonuleotides are of sufficient length that they will hybridize under stringent conditions only with a RNA/cDNA for that gene to which they correspond, the oligo can be used to identify the EST and in turn the clone from which it was derived and by subsequent cloning, obtain the sequence of the full-length cDNA and its genomic counterparts, i.e., the gene, from which it was obtained.

In other words, the ESTs identified by the method of this invention can be employed to determine the complete sequence of the mRNA, in the form of transcribed cDNA, by using the EST as a probe to identify a cDNA clone corresponding to a full-length transcript, followed by sequencing of that clone. The EST or the full length cDNA clone can also be used as a probe to identify a genomic clone or clones that contain the complete gene including regulatory and promoter regions, exons, and introns.

It should be appreciated that one does not have to be restricted in using ESTs from a particular tissue from which probe RNA or cDNA is obtained, rather any or all ESTs (known or unknown) may be placed on the support. Hybridization will be used a form diagnostic patterns or to identifiy which particular EST is detected. For example, all known ESTs from an organism are used to produce a "master" solid support to which control sample and disease samples are alternately hybridized. One then detects a pattern of hybridization associated with the particular disaease state which then forms the basis of a diagnostic test or the isolation of disease specific ESTs from which the intact gene may be cloned and sequenced leading uiltimately to a defined therapuetic target.

Methods for obtaining complete gene sequences from ESTs are well-known to those of skill in the art. See, generally, Sambrook et al, cited above. Briefly, one suitable method involves purifying the DNA from the clone that was sequenced to give the EST and labeling the isolated insert DNA. Suitable labeling systems are well known to those of skill in the art [see, eg. Basic Methods in Molecular Biology, L. G. Davis et al, ed., Elsevier Press, NY (1986)]. The labeled EST insert is then used as a probe to screen a lambda phage cDNA library or a plasmid cDNA library, identifying colonies containing clones related to the probe cDNA which can be purified by known methods. The ends of the newly purified clones are then sequenced to identify full length sequences and complete sequencing of full length clones is performed by enzymatic digestion or primer walking. A similar screening and clone selection approach can be applied to clones from a genomic DNA library.

Additionally, an EST or gene identified by this method as associated with inherited disorders can be used to determine at what stage during embryonic development the selected gene from which it is derived is developed by screening embryonic DNA libraries from various stages of development, e.g. 2-cell, 8-cell, etc., for the selected gene. As has been mentioned above, the invention may be applied in addtional temporal modes for monitoring the progression of a disease state, the efficacy of a particular treatment modality or the aging process of an individual.

Thus, the methods of this invention permit the identification, isolation and sequencing of a gene which is differentially expressed in a selected disease/infection. As described in more detail below, the identified gene may then be employed to obtain any protein encoded thereby, or may be employed as a target for diagnostic methods or therapeutic approaches to the treatment of the disease, including, e.g., drug development.

The same methods as described above for the identification of genes, including genes of unknown function, which are differentially expressed in a disease state, may also be employed to identify other genes of interest. For example, another embodiment of this invention includes a method for identifying a gene of a pathogen which is expressed in a biological sample of an animal infected with that pathogen or the gene of the host which is altered in its expression as a result of the infection.

One such method employs a healthy test surface as described above, employing defined oligonucleotide/polynucleotides from a sample of a healthy, uninfected animal. The second such surface has immobilized at pre-defined regions thereon a plurality of defined oligonucleotide/polynucleotide sequences of ESTs isolated from at least one analogous tissue or cell sample of an infected animal (the "infection test surface"). Polynucleotide sequences are isolated from a biological sample from a healthy animal ("healthy control") and a second sample is similarly prepared from an animal infected with the selected pathogen ("infection sample"). These two samples are desirably selected from the cell or tissue analogous to that which provided the immobilized oligonucleotide/polynucleotides. It would also be possible to provide samples from the nucleic acid of the pathogen itself.

According to the method the healthy control sample is contacted with one set of the healthy test surface and the infection test surface described above for a time sufficient to permit detectable hybridization to occur between the sample and the immobilized defined oligonucleotide/polynucleotides on each surface. The results of this hybridization are a first hybridization pattern formed between the nucleotides of healthy control and the healthy test surface and a second hybridization pattern formed between the nucleotides of healthy control sample and the infection test surface.

In a similar manner, the infection sample is detectably hybridized to another set of healthy test and infection test surfaces, forming a third hybridization pattern between the infection sample and healthy test surface and a fourth hybridization pattern between the infection sample and the infection test surface.

Comparing the four hybridization patterns permits detection of those defined oligonucleotide/polynucleotides which are differentially expressed between the healthy animal and the animal infected with the pathogen by the presence of differences in the hybridization patterns at pre-defined regions. As mentioned differential expression is not required and simple qualitative analysis is possible by reference to gene expression which is simply present or absent.

A second embodiment of this method parallels the second embodiment of the method as applied to disease above, i.e., the same process is employed, with the exception that plurality of defined oligonucleotide/polynucleotide sequences forming the healthy test sample surface and the infection test sample surface are immobilized on a single solid support. The resulting first hybridization pattern (healthy control sample with healthy/infection test sample) and second hybridization pattern (infection sample with healthy/infection test sample) permits detection of those defined oligonucleotide/polynucleotides which are differentially expressed between the healthy control and the infection sample by the presence of differences in the hybridization patterns at pre-defined regions. The oligonucleotide/polynucleotides on each surface which correspond to the pattern differences may be readily identified with the corresponding ESTs from which the oligonucleotide/polynucleotides are obtained.

As described above for the methods for identifying differential gene expression between diseased and healthy animals, the oligonucleotide/polynucleotides on each surface which correspond to the pattern differences may be readily identified with the corresponding ESTs from which the oligonucleotide/polynucleotide sequences are obtained and the genes expressed by the pathogen identified for similar purposes. Other embodiments of these methods may be developed with resort to the teaching herein, by altering the samples which provide the defined oligonucleotide/polynucleotides. For example, an EST, identified with a differentially expressed gene by the method of this invention is also useful in detecting genes expressed in the various stages of an pathogen's development, particularly the infective stage and following the cours of drug treatment and emergence of resistant variants. For example, employing the techniques described above, the EST can be used for detecting a gene in various stages of the parasitic *Plasmodium* species life cycle, which include blood stages, liver stages, and gametocyte stages.

### B. Diagnostic Methods

In addition to use of the methods and compositions of this invention for identifying differentially expressed genes, another embodiment of this invention provides diagnostic methods for diagnosing a selected disease state, or a selected state resulting from aging, exposure to drugs or infection in an animal. According to this aspect of the invention, a first surface, described as the healthy test surface above, and a second surface, described as the disease test surface or infection test surface, are prepared depending on the disease or infection to be diagnosed. The same processes of detectable hybridization to a first and second set of these surfaces with the healthy control sample and disease/infection sample are followed to provide the four above-described hybridization patterns, i.e., healthy control sample with healthy test surface; healthy control sample with disease/infection test surface; disease/infection sample with healthy test surface; and disease/infection sample with disease/infection test surface.

The diagnosis of disease or infection is provided by comparing the four hybridization patterns. Substantial differences between the first and third hybridization patterns, respectively, and the second and fourth hybridization patterns, respectively, indicate the presence of the selected disease or infection in said animal. Substantial similarities in the first and third hybridization patterns and second and fourth hybridization patterns indicates the absence of disease or infection.

A similar embodiment utilizes the single surface bearing both the healthy test surface defined oligonucleotide/polynucleotides and the disease/infection test surface defined oligonucleotide/polynucleotides as described above. Parallel process steps as described above for detection of genes differentially expressed in disease and infected states are followed, resulting in a first hybridization pattern (healthy control sample with single healthy and disease/infection test sample) and a second hybridization pattern (disease/infection sample with another copy of the single healthy and disease/infection test sample).

Diagnosis is accomplished by comparing the two hybridization patterns, wherein substantial differences between the first and second hybridization patterns indicate the presence of the selected disease or infection in the animal being tested. Substantially similar first and second hybridization patterns indicate the absence of disease or infection. This like many of the foregoing embodiments may use known or unknown ESTs derived from many libraries.

### C. Other Methods of the Invention

As is obvious to one of skill in the art upon reading this disclosure, the compositions and methods of this invention may also be used for other similar purposes. For example, the general methods and compositions may be adapted easily by manipulation of the samples selected to provide the standardized defined oligonucleotide/polynucleotides, and selection of the samples selected for hybridization thereto. One such modification is the use of this invention to identify cell markers of any type, e.g., markers of cancer cells, stem cell markers, and the like. Another modification involves the use of the method and compositions to generate hybridization patterns useful for forensic identification or an 'expression fingerprint' of genes for identification of one member of a species from another. Similarly, the methods of this invention may be adapted for use in tissue matching for transplantation purposes as well as for molecular histology, i.e., to enable diagnosis of disease or disorders in pathology tissue samples such as biopsies. Still another use of this method is in monitoring the effects of development and aging upon the gene expression in a selected animal, by preparing surfaces bearing oligonucleotide/polynucleotides prepared from samples of standardized younger members of the species being tested. Additionally the patient can serve as an internal control by virtue of having the method applied to blood samples every 5-10 years during his lifetime.

Still another intriguing use of this method is in the area of monitoring the effects of drugs on gene expression, both in laboratories and during clinical trials with animal, especially humans. Because the method can be readily adapted by altering the above parameters, it can essentially be employed to identify differentially expressed genes of any organism, at any stage of development, and under the influence of any factor which can affect gene expression.

### IV. The Genes and Proteins Identified

Application of the compositions and methods of this invention as above described also provide other compositions, such as any isolated gene sequence which is differentially expressed between a normal healthy animal and an animal having a disease or infection. Another embodiment of this invention is any isolated pathogen gene sequence which is expressed in tissue or cell samples of an infected animal. Similarly an embodiment of this invention is any gene sequence identified by the methods described herein.

These gene sequences may be employed in conventional methods to produce isolated proteins encoded thereby. To produce a protein of this invention, the DNA sequences of a desired gene identified by the use of the methods of this invention or portions thereof are inserted into a suitable expression system. Desirably, a recombinant molecule or vector is constructed in which the polynucleotide sequence encoding the protein is operably linked to a heterologous expression control sequence permitting expression of the human protein. Numerous types of appropriate expression vectors and host cell systems are known in the art for mammalian (including human) expression, insect, e.g., baculovirus expression, yeast, fungal, and bacterial expression, by standard molecular biology techniques.

The transfection of these vectors into appropriate host cells, whether mammalian, bacterial, fungal, or insect, or into appropriate viruses, can result in expression of the selected proteins. Suitable host cells or cell lines for transfection, and viruses, as well as methods for the construction and transfection of such host cells and viruses are well-known. Suitable methods for transfection, culture, amplification, screening, and product production and purification are also known in the art.

The genes and proteins identified by this invention can be employed, if desired in diagnostic compositions useful for the diagnosis of a disease or infection using conventional diagnostic assays. For example, a diagnostic reagent can be developed which detectably targets a gene sequence or protein of this invention in a biological sample of an animal. Such a reagent may be a complementary nucleotide sequence, an antibody (monoclonal, recombinant or polyclonal), or a chemically derived agonist or antagonist. Alternatively, the proteins and polynucleotide sequences of this invention, fragments of same, or complementary sequences thereto, may themselves be useful as diagnostic reagents for diagnosing disease states with which the ESTs of the invention are associated. These reagents may optionally be labelled using diagnostic labels, such as radioactive labels, colorimetric enzyme label systems and the like conventionally used in diagnostic or therapeutic methods, e.g, Northern and Western blotting, antigen-antibody binding and the like. The selection of the appropriate assay format and label system is within the skill of the art and may readily be chosen without requiring additional explanation by resort to the wealth of art in the diagnostic area.

Additionally, genes and proteins identified according to this invention may be used therapeutically. For example, the EST-containing gene sequences may be useful in gene therapy, to provide a gene sequence which in a disease is not properly or sufficiently expressed. In such a method, a selected gene sequence of this invention is introduced into a suitable vector or other delivery system for delivery to a cell containing a defect in the selected gene. Suitable delivery systems are well known to those of skill in the art and enable the desired EST or gene to be incorporated into the target cell and to be translated by the cell. The EST or gene sequence may be introduced to mutate the existing gene by recombination or provide an active copy thereof in addition to the inactive gene to replace its function.

Alternatively, a protein encoded by an EST or gene of the invention may be useful as a therapeutic reagent for delivery of a biologically active protein, particularly when the disease state is associated with a deficiency of this protein. Such a protein may be incorporated into an appropriate therapeutic formulation, alone or in combination with other active ingredients. Methods of formulating such therapeutic compositions, as well as suitable pharmaceutical carriers, and the like, are well known to those of skill in the art. Still an additional method of delivering the missing protein encoded by an EST, or the gene from which a selected EST was derived, involves expressing it directly *in vivo.* Systems for such *in vivo* expression are well known in the art.

Yet another use of the ESTs, genes identified according to the methods of this invention, or the proteins encoded thereby is a target for the screening and development of natural or synthetic chemical compounds which have utility as therapeutic drugs for the treatment of disease states associated with the identified genes and ESTs derived therefrom. As one example, a compound capable of binding to such a protein encoded by such a gene and either preventing or enhancing its biological activity may be a useful drug component for the treatment or prevention of such disease states.

Conventional assays and techniques may be used for the screening and development of such drugs. As one example, a method for identifying compounds which specifically bind to or inhibit or activate proteins encoded by these gene sequences can include simply the steps of contacting a selected protein or gene product, with a test compound to permit binding of the test compound to the protein; and determining the amount of test compound, if any, which is bound to the protein. Such a method may involve the incubation of the test compound and the protein immobilized on a solid support. Still other conventional methods of drug screening can involve employing a suitable computer program to determine compounds having similar or complementary chemical structures to that of the gene product or portions thereof and screening those compounds either for competitive binding to the protein to detect enhanced or decreased activity in the presence of the selected compound.

Thus, through use of such methods, the present invention is anticipated to provide compounds capable of interacting with these genes, ESTs, or encoded proteins, or fragments thereof, and either enhancing or decreasing the biological activity, as desired. Such compounds are believed to be encompassed by this invention.

Numerous modifications and variations of the present invention are included in the above-identified specification and are expected to be obvious to one of skill in the art. Such modifications and alterations to the compositions and processes of the present invention are believed to be encompassed in the scope of the claims appended hereto.

## Claims

1. A method for identifying genes which are differentially expressed in two different pre-determined states of an organism comprising:
a. providing a first surface on which is immobilized at pre-defined regions on said surface a plurality of defined oligonucleotide/polynuclevtide sequences, each sequence selected from the group consisting of a fragment of an EST, an entire EST a fragment of a gene or an entire gene, isolated from a DNA library prepared from at least one selected cell, tissue, organ or organism sample in a first state and present in excess relative to the polynucleotide to be hybridized;
b. providing a second surface on which is immobilized at pre-defined regions on said surface a plurality of defined oligonucleotide/polynucleotide sequences, each sequence selected from the group consisting of a fragment of an EST, an entire EST a fragment of a gene or an entire gene, isolated from a DNA library prepared from at least one selected cell, tissue, organ or organism sample in a second state and present in excess relative to the polynucleotide to be hybridized;
c. detectably hybridizing to a set of said first and second surfaces polynucleotide sequences isolated from a sample from a said organism in said first state, said sample selected from sources analogous to the sources of step (a), said hybridization sufficient to form a first and second hybridization pattern on each said first and second surface,
d. detectably hybridizing to a set of said first and second surfaces polynucleotide sequences isolated from a sample from said organism in said second state, said sample selected from sources analogous to the sources of step (c), said hybridization sufficient to form a third and fourth hybridization pattern on each said first and second surface,
e. comparing at least two of the four hybridization patterns, wherein genes differentially expressed in said first and second states are identified by the presence of differences in the hybridization patterns at pre-defined regions;
f. identifying the oligonucleotide/polynucleotides on each surface which correspond to said pattern differences and the corresponding ESTs or larger gene fragment from which the oligonucleotide/polynucleotides were obtained, whereby identification of the EST or larger gene fragment permits identification of the gene from which the ESTs or larger gene fragment were derived.

2. The method according to Claim 1 wherein said first and second states are respectively healthy and disease; pathogen uninfected and pathogen infected; a first progression state and a second progression of a disease or infection; a first treatment state and a second treatment state of a disease or infection; or a first developmental and a second developmental state.

3. The method according to Claim 1 wherein said organism is a plant or an animal.

4. The method according to Claim 3 wherein said aniaml is a human.

5. A method for identifying genes which are differentially expressed in a normal healthy animal and an animal having a disease comprising:
a. providing a first surface on which is immobilized at pre-defined regions on said surface a plurality of defined oligonucleotide/polynucleotide sequences, each sequence each sequence selected from the group consisting of a fragment of an EST, an entire EST a fragment of a gene or an entire gene, isolated from a DNA library prepared from at least one selected cell, tissue, organ or organism sample in a healthy animal and present in excess relative to the polynucleotide to be hybridized;
b. providing a second surface on which is immobilized at pre-defined regions of said surface a plurality of defined oligonucleotide/polynucleotide sequences, each sequence each sequence selected from the group consisting of a fragment of an EST, an entire EST a fragment of a gene or an entire gene, isolated from a DNA library prepared from at least one selected cell, tissue, organ or organism sample from an animal having said disease and present in excess relative to the polynucleotide to be hybridized;
c. detectably hybridizing to a set of said first and second surfaces polynucleotide sequences isolated from a sample from a healthy animal, said sample selected from sources analogous to the sources of step (a), said hybridization sufficient to form a first and second hybridization pattern on each said first and second surface, said sample selected from a cell or tissue sample analogous to the sample of step (a), said hybridization sufficient to form a first and second hybridization pattern on each said first and second surface;
d. detectably hybridizing to a set of said first and second surfaces polynucleotide sequences isolated from a sample from an animal having said disease, said sample selected from a cell or tissue sample analogous to the sample of step (c), said hybridization sufficient to form a third and fourth hybridization pattern on each said first and second surface,
e. comparing at least two of the four hybridization patterns, wherein genes differentially expressed in said first and second states are identified by the presence of differences in the hybridization patterns at pre-defined regions;
f. identifying the oligonucleotide/polynucleotides on each surface which correspond to said pattern differences and the corresponding ESTs or larger gene fragment from which the oligonucleotide/polynucleotides were obtained, whereby identification of the EST or larger gene fragment permits identification of the gene from which the ESTs or larger gene fragment were derived.

6. A method for identifying genes which are differentially expressed in a normal healthy animal and an animal having a disease comprising:
a. providing a surface on which is immobilized at pre-defined regions on said surface a plurality of defined oligonucleotide/polynucleotide sequences, each sequence selected from the group consisting of a fragment of an EST, an entire EST a fragment of a gene or an entire gene isolated from a DNA library prepared from the group selected from at least one selected cell, tissue, organ or organism sample in of a healthy animal and an analogous selected sample of an animal having said disease and both present in excess relative to the polynucleotide to be hybridized;
b. detectably hybridizing to a first copy of said surface polynucleotide sequences isolated from a healthy animal, said sample selected from a cell or tissue sample analogous to the sample of step (a), said hybridization sufficient to form a first hybridization pattern on said surface;
c. detectably hybridizing to a second copy of said surface polynucleotide sequences isolated from an animal having said disease, said sample selected from a cell or tissue sample analogous to the sample of step (a), said hybridization sufficient to form a second hybridization pattern on said surface;
d. comparing the two hybridization patterns, wherein genes differentially expressed in a disease state are identified by the presence of differences in the hybridization patterns at pre-defined regions;
e. identifying the oligonucleotide/polynucleotides on each surface which correspond to said pattern differences and the corresponding ESTs from which the oligonucleotide/polynucleotides are obtained, whereby identification of the EST permits identification of the gene from which the ESTs were derived.

7. A method for identifying a gene of a pathogen which is expressed in a biological sample of an animal infected with said pathogen comprising:
a. providing a first surface on which is immobilized at pre-defined regions on said surface a plurality of defined oligonucleotide/polynucleotide sequences, each sequence selected from the group consisting of a fragment of an EST, an entire EST a fragment of a gene or an entire gene isolated from a DNA library prepared from at least one selected cell, tissue, organ or organism sample of a healthy, uninfected animal and present in excess relative to the polynucleotide to be hybridized;
b. providing a second surface on which is immobilized at pre-defined regions of said surface a plurality of defined oligonucleotide/polynucleotide sequences, each sequence selected from the group consisting of a fragment of an EST, an entire EST a fragment of a gene or an entire gene isolated from at least one selected cell, tissue, organ or organism sample of an infected animal;
c. detectably hybridizing to a set of said first and second surfaces polynucleotide sequences isolated from a sample from a healthy animal, said sample selected from a cell or tissue sample analogous to the sample of step (a), said hybridization sufficient to form first and second hybridization patterns on each said first and second surface,
d. detectably hybridizing to a set of said first and second surfaces polynucleotide sequences isolated from a sample from an infected animal, said sample selected from a cell or tissue sample analogous to the sample of step (a), said hybridization sufficient to form third and fourth hybridization patterns on each said first and second surface,
e. comparing the four hybridization patterns, wherein genes of said pathogen which are expressed in an infected animal are identified by the presence of differences in the hybridization patterns at pre-defined regions;
f. identifying the oligonucleotide/polynucleotides on each surface which correspond to said pattern differences and the corresponding ESTs from which the oligonucleotide/polynucleotides are obtained, whereby identification of the EST permits identification of the gene from which the ESTs were derived.

8. A method for identifying a gene of a pathogen which is expressed in a biological sample of an animal infected with said pathogen comprising:
a. providing a surface on which is immobilized at pre-defined regions on said surface a plurality of defined oligonucleotide/polynucleotide sequences, each sequence selected from the group consisting of a fragment of an EST, an entire EST a fragment of a gene or an entire gene isolated from a DNA library prepared from the group selected from at least one selected cell, tissue, organ or organism sample in of a healthy animal and an analogous selected sample of an animal having said disease and both present in excess relative to the polynucleotide to be hybridized
b. detectably hybridizing to a first copy of said surface polynucleotide sequences isolated from a sample from a healthy animal, said sample selected from a cell or tissue sample analogous to the sample of step (a), said hybridization sufficient to form a first hybridization pattern on said surface;
c. detectably hybridizing to a second copy of said surface polynucleotide sequences isolated from a sample from an infected animal, said sample selected from a cell or tissue sample analogous to the sample of step (a), said hybridization sufficient to form a second hybridization pattern on said surface;
d. comparing the two hybridization patterns, wherein genes of said pathogen which are expressed in an infected animal are identified by the presence of differences in the hybridization patterns at pre-defined regions;
e. identifying the oligonucleotide/polynucleotides on each surface which correspond to said pattern differences and the corresponding ESTs from which the oligonucleotide/polynucleotides are obtained, whereby identification of the EST permits identification of the gene from which the ESTs were derived.

9. A composition suitable for use in hybridization comprising a solid surface on which is immobilized at pre-defined regions on said surface a plurality of defined oligonucleotide/polynucleotide sequences for hybridization, each sequence selected from the group consisting of a fragment of an EST, an entire EST a fragment of a gene or an entire gene isolated from a DNA library prepared from the group selected from at least one selected cell, tissue, organ or organism sample of a healthy animal, at least one analogous sample of said animal having a disease, at least one analogous sample of said animal infected with a microbial pathogen, and any combination thereof.

10. An isolated gene sequence which is differentially expressed in a normal healthy animal and an animal having a disease, identified by the method of claim 1.

11. An isolated pathogen gene sequence which is expressed in tissue or cell samples of an infected animal identified by the method of claim 7.

12. A diagnostic composition useful for the diagnosis of a disease comprising a reagent capable of detectably targeting a gene sequence of claim 10 in a biological sample of an animal.

13. A diagnostic composition useful for the diagnosis of infection by a pathogen comprising a reagent capable of detectably targeting a gene sequence of claim 11 in a biological sample of an animal.

14. An isolated protein produced by expression of a gene sequence of claim 10.

15. An isolated pathogen protein produced by expression of a gene sequence of claim 11.

16. A therapeutic composition comprising a protein or fragment thereof selected from the group consisting of a protein of claim 10 and a protein of claim 15.

17. A method for diagnosing a selected disease or infection in an animal comprising:
a. providing a first surface on which is immobilized at pre-defined regions on said surface a plurality of defined oligonucleotide/polynucleotide sequences, each sequence selected from the group consisting of a fragment of an EST, an entire EST a fragment of a gene or an entire gene, isolated from a DNA library prepared from at least one selected cell, tissue, organ or organism sample of a healthy animal and present in excess relative to the polynucleotide to be hybridized;
b. providing a second surface on which is immobilized at pre-defined regions of said surface a plurality of defined oligonucleotide/polynucleotide sequences, each sequence comprising a fragment of an EST isolated from at least one said tissue of an animal having said disease;
c. detectably hybridizing to a set of said first and second surfaces polynucleotide sequences isolated from a DNA library prepared from a sample from a healthy animal, said sample selected from a cell or tissue sample analogous to the sample of step (a), said hybridization sufficient to form a first and second hybridization pattern on each said first and second surface;
d. detectably hybridizing to a set of said first and second surfaces polynucleotide sequences isolated from a DNA library prepared from a sample from an animal having said disease, said sample selected from a cell or tissue sample analogous to the sample of step (c), said hybridization sufficient to form a third and fourth hybridization pattern on each said first and second surface;
e. comparing the four hybridization patterns, wherein substantial differences between the first and third hybridization patterns and the second and fourth hybridization patterns indicates the presence of said selected disease or infection in said animal, and substantial similarities in said first and third hybridization patterns and second and fourth hybridization patterns indicates the absence of disease or infection.

18. A method for diagnosing a selected disease or infection in an animal comprising:
a. providing a surface on which is immobilized at pre-defined regions on said surface a plurality of defined oligonucleotide/polynucleotide sequences, each sequence comprising a fragment of an EST isolated from a DNA library prepared from the group consisting of a selected cell or tissue sample of a healthy animal and an analogous selected cell or tissue sample of an animal having said disease;
b. detectably hybridizing to a first copy of said surface polynucleotide sequences isolated from a sample from a healthy animal, said sample selected from a cell or tissue sample analogous to the sample of step (a), said hybridization sufficient to form a first hybridization pattern on said surface;
c. detectably hybridizing to a second copy of said surface polynucleotide sequences isolated from a DNA library prepared from a sample from an animal having said disease, said sample selected from a cell or tissue sample analogous to the sample of step (a), said hybridization sufficient to form a second hybridization pattern on said surface;
d. comparing the two hybridization patterns, wherein substantial differences between the first and second hybridization patterns indicates the presence of said selected disease or infection in said animal, and substantial similarities in said first and second hybridization patterns indicates the absence of disease or infection.
